# EUROPÄISCHE PATENTSCHRIFT

(11) **EP 3 717 052 B1**
(45) Veröffentlichungstag und Bekanntmachung des Hinweises auf die Patenterteilung: **08.09.2021**
(21) Anmeldenummer: 18782922.1
(22) Anmeldetag: 28.09.2018
(51) Int. Cl.: A61M 25/02

(54) **KANÜLENFIXIERPFLASTER**
FIXATION PLASTER FOR CANNULA
EMPLÂTRE DE FIXATION POUR CANULE

(30) Priorität: 29.11.2017 DE 102017128321
(43) Veröffentlichungstag der Anmeldung: 07.10.2020
(73) Patentinhaber: Lohmann & Rauscher GmbH & Co. KG, 56567 Neuwied (DE)
(72) Erfinder: DODEL, Kirsten, 56566 Neuwied (DE); KUPPERT, Steven, 56645 Nickenich (DE); SASTGES, Thomas, 56626 Andernach (DE); ALBUS, Sascha, 56581 Melsbach (DE); SIECKENDIECK, Sandra, 56567 Neuwied (DE)
(74) Vertreter: Seranski, Klaus
(86) Internationale Anmeldenummer: PCT/EP2018/076424
(87) Internationale Veröffentlichungsnummer: WO 2019/105631

(56) Entgegenhaltungen:
- WO-A1-2014/099709
- WO-A2-2014/039891
- DE-U1-202007 007 634
- DE-U1-202007 007 634
- US-A- 6 124 521

## Beschreibung

Die Erfindung betrifft ein Pflaster zum Fixieren von Kanülen nach dem Oberbegriff den Patentanspruchs 1.

Derartige Pflaster sind beispielsweise in der DE 20 2007 007 634 U1 beschrieben. Dabei ist die Trägeranordnung aus einer Vliesschicht gebildet, welche einerseits mit einem Haftmittel beschichtet ist und andererseits von einer die Barrierelage bildenden transparenten Polyurethanfolie abgedeckt ist. Die bekannten Pflaster weisen eine von der Trägeranordnung vollständig umlaufende Beobachtungsöffnung auf, welche von der Polyurethanfolie überdeckt ist. Die Beobachtungsöffnung ermöglicht eine Sichtkontrolle der Kanüleneinstichstelle während und nach Applikation des Pflasters.

Die bekannten Pflaster weisen weiter eine die Trägeranordnung und die Barrierelage durchsetzende Ausnehmung 28 auf, welche von dem Kanülenschlauch durchsetzt wird, so dass ein Teil des Pflasters nach der Applikation zwischen Kanüle und Haut angeordnet ist, während der andere Teil des Pflasters die Kanüle und die Kanüleneinstichstelle mitüberdeckt, wobei die Kanüleneinstichstelle durch die Beobachtungsöffnung weiter sichtbar bleibt.

Wenngleich die Applikation der bekannten Pflaster vergleichsweise einfach ist und eine Sichtkontrolle der Kanüleneinstichstelle ermöglicht, hat es sich gezeigt, dass bei Einsatz der bekannten Pflaster in einigen Fällen ein unbeabsichtigtes Lösen der Kanüle aus der Haut erfolgt und/oder eine Infektion der Kanüleneinstichstelle beobachtet wird. Ähnliche Probleme werden auch bei Pflastern gemäß WO 2014/005140 A1 beobachtet, bei denen ein zusätzlicher Fixierstreifen vorgesehen ist.

Bei einer in der WO 2014/03989 A1 beschriebene Anordnung zum Fixieren von Kanülen ist ein mit einer Ausnehmung zum Aufnehmen einer Schraubverbindung der Kanülenanordnung ausgestattetes Retainer Pad vorgesehen, das von einer transparenten Folie abgedeckt sein kann. Weitere Pflaster zum Fixieren von Kanülen sind in der WO 2014/099709A1 und der US 6,124,521 A angegeben.

Angesichts dieser Probleme im Stand der Technik liegt der Erfindung die Aufgabe zugrunde, ein Pflaster zum Fixieren von Kanülen bereitzustellen, welches einerseits eine zuverlässige Kanülenfixierung ermöglicht und andererseits das Infektionsrisiko zu reduzieren vermag.

Erfindungsgemäß wird diese Aufgabe durch die im kennzeichnenden Teil des Patentanspruchs 1 angegebene Weiterbildung der bekannten Pflaster zum Fixieren von Kanülen gelöst.

Die Erfindung geht auf die Erkenntnis zurück, dass mit den bekannten Pflastern zum Fixieren von Kanülen auf den die Ausnehmung in der Trägeranordnung und der Barrierelage durchsetzenden Kanülenschlauch wirkende Zugkräfte nur unzureichend aufgenommen werden können, was zu einem unbeabsichtigten Lösen der Kanüle führen kann. Ferner bildet die Ausnehmung in der Barrierelage, welche zum Durchführen des Kanülenschlauchs bei den bekannten Pflastern benötigt wird, eine Schwachstelle, durch welche Bakterien zur Kanüleneinstichstelle gelangen und Infektionen verursachen können.

Bei erfindungsgemäßen Pflastern wird die im Allgemeinen stegförmige Anfasshilfe der Kanülen, welche bei der Applikation bekannter Pflaster eher störend ist, nutzbar gemacht. Dazu wird die Trägeranordnung mit einer zum Aufnehmen dieser Anfasshilfe ausgelegten Aufnahme ausgestattet. Die Aufnahme wird von der Haftfläche der Trägeranordnung umgeben. Mit der Aufnahme können auf die Kanüle und damit auch auf die Anfasshilfe wirkende Zugkräfte aufgenommen und an die Haftfläche weitergegeben werden. Dadurch erfolgt eine Zugentlastung im Bereich der Kanüleneinstichstelle und eine sichere Fixierung der Kanüle. Die Aufnahme wird von der Barrierelage überdeckt. Dadurch wird dem Eindringen von Bakterien in den Bereich der Kanüleneinstichstelle entgegengewirkt und eine zusätzliche Fixierung erreicht.

Überraschenderweise hat es sich gezeigt, dass schon durch die vergleichsweise dünne, im Allgemeinen als Vliesschicht verwirklichte Trägeranordnung eine ausreichende Zugkraftentlastung über die in der Aufnahme aufgenommene Anfasshilfe erfolgen kann, wobei die Barrierelage im Allgemeinen nachgiebig ist, so dass im Bereich der in der Aufnahme aufgenommenen Anfasshilfe eine Ausbuchtung der auch im Rahmen der Erfindung vorzugsweise in Form einer transparenten Polyurethanfolie ausgeführten Barrierelage erfolgt.

Im Hinblick auf den Umstand, dass die Anfasshilfe herkömmlicher Kanülen im Allgemeinen stegförmig ausgeführt ist, ist vorgesehen, dass die die Trägeranordnung durchsetzende Aufnahme dafür einen etwa geradlinig verlaufenden Aufnahmeschlitz in der vorzugsweise als Vliesschicht ausgeführten Trägeranordnung aufweist, insbesondere als geradlinig verlaufender Schlitz ausgeführt ist, dessen Länge an die Abmessungen der Anfasshilfe der Kanüle angepasst sein kann. Die Länge des Schlitzes beträgt allerdings im Hinblick auf eine ausreichende Krafteinleitung der auf die Kanüle einwirkenden Zugkräfte in den Bereich der Haftfläche vorzugsweise weniger als 50 %, insbesondere weniger als 25 % der Breite der Trägeranordnung. Zur Vermeidung des Ausreißens des Aufnahmeschlitzes bei auf den Kanülenschlauch bzw. die Anfasshilfe einwirkenden Zugkräften hat es sich allerdings als zweckmäßig erwiesen, wenn die Länge des Schlitzes mehr als 7 %, vorzugsweise mehr als 12 % der Breite der Trägeranordnung im Bereich des Schlitzes beträgt. Bei bevorzugten Ausführungsformen der Erfindung beträgt die Länge des Aufnahmeschlitzes zwischen 4 und 15 mm, vorzugsweise zwischen 6 und 13 mm, insbesondere etwa 8 mm.

Im Hinblick auf die Vermeidung von Schwachstellen im Bereich der Enden des Aufnahmeschlitzes hat es sich als zweckmäßig erwiesen, wenn die Breite des Schlitzes in einer senkrecht zu dessen Längsrichtung verlaufenden Richtung mehr als 10 %, vorzugsweise mehr als 20 % der Schlitzlänge beträgt. Zur Sicherstellung einer ausreichenden Arretierung einer stegförmigen Anfasshilfe in dem Aufnahmeschlitz hat es sich aber als zweckmäßig erwiesen, wenn die Breite des Schlitzes weniger als 35 % der Schlitzlänge beträgt. Bei bevorzugten Ausführungsformen der Erfindung beträgt die Schlitzbreite etwa 0,5 bis 5 mm, vorzugsweise etwa 1,5 bis 3,5 mm, besonders bevorzugt etwa 2 mm.

Ein erfindungsgemäßes Kanülenfixierpflaster überdeckt die Kanülenanordnung ohne von dieser durchsetzt zu werden. Zum Anmodellieren der Trägeranordnung an die Kanüle im Sinne einer zuverlässigen Kanülenfixierung ist im Rahmen der Erfindung vorgesehen, dass eine sich ausgehend von einem vorzugsweise etwa parallel zur Längsrichtung des Schlitzes verlaufenden Rand der Trägeranordnung in Richtung auf die Aufnahme erstreckende Einbuchtung vorgesehen ist, die sich vorzugsweise von einer die Länge des Schlitzes übersteigenden Breite im Bereich des Rands in Richtung auf den Schlitz verjüngt und an ihrem dem Schlitz zugewandten Ende eine geringere Breite aufweist als der Schlitz selbst.

Dabei kann die Breite der Einbuchtung im Bereich des Rands etwa 10 bis 20 mm, vorzugsweise etwa 12 bis 16 mm, insbesondere etwa 14 mm aufweisen. An ihrem dem Aufnahmeschlitz zugewandten Ende kann die Einbuchtung zur Vermeidung von das Einreißen begünstigenden Spitzen abgerundet ausgeführt sein, wobei eine an den Scheitel der Abrundung angelegte Tangente etwa parallel zur Längsrichtung des Schlitzes verläuft. Zwischen dem Aufnahmeschlitz und dem Scheitel der Einbuchtung ist vorzugsweise ein Streifen der Trägeranordnung mit einer Breite von mehr als 1 mm, insbesondere etwa 3 mm vorgesehen.

Ähnlich wie bei den aus der DE 20 2007 007 634 U1 bekannten Kanülenfixierpflastern, weist die Trägeranordnung erfindungsgemäßer Pflaster eine von der Trägeranordnung nach Art eines Rahmens insbesondere vollständig umlaufene und durch einen Rahmenabschnitt der Trägeranordnung von der Aufnahme getrennte, vorzugsweise auf der der Haftfläche abgewandten Seite der Trägeranordnung von der Barrierelage überdeckte Beobachtungsöffnung auf. Dabei ist die Beobachtungsöffnung in einer senkrecht zum Aufnahmeschlitz verlaufenden Richtung von dem Aufnahmeschlitz beabstandet, wobei der Aufnahmeschlitz zwischen der Beobachtungsöffnung und der Einbuchtung angeordnet sein kann. Der Abstand zwischen Aufnahmeschlitz und Beobachtungsöffnung, also die Breite des Rahmenabschnitts, beträgt vorzugsweise mehr als 1 mm, insbesondere etwa 3 mm.

Bei einer besonders bevorzugten Ausführungsform der Erfindung ist die Breite der Trägeranordnung in einer parallel zum Aufnahmeschlitz verlaufenden Richtung im Bereich der Beobachtungsöffnung geringer als die Breite der Trägeranordnung im Bereich der Einbuchtung und/oder der Aufnahme. So wird im Bereich der Einbuchtung bzw. der Aufnahme ein das Fixieren der Kanüle begünstigender Fixierabschnitt außerhalb der Beobachtungsöffnung und mit Abstand von der Kanüleneinstichstelle geschaffen.

Herstellungstechnisch und im Sinne einer einfachen Applikation hat es sich als günstig erwiesen, wenn die Trägeranordnung insgesamt etwa spiegelsymmetrisch bezüglich einer vorzugsweise etwa senkrecht zum Aufnahmeschlitz verlaufenden Symmetrieebene angeordnet ist.

Wie vorstehend bereits angesprochen, kann die Barrierelage ebenso wie bei Kanülenfixierpflastern nach dem bekannten Stand der Technik auch bei erfindungsgemäßen Kanülenfixierpflastern eine transparente Polyurethanfolie aufweisen. Dabei ist die Barrierelage zweckmäßigerweise zumindest im Bereich der Aufnahme und/oder der Beobachtungsöffnung mit einem das Fixieren des Pflasters an der Haut und/oder der Anfasshilfe fördernden Haftmittel ausgestattet.

Ebenso wie bei herkömmlichen Pflastern kann ein erfindungsgemäßes Kanülenfixierpflaster eine lösbar an der Haftfläche angeordnete, vorzugsweise mehrteilige Abdeckanordnung, beispielsweise aus Silikonpapier, aufweisen, welche zweckmäßigerweise die gesamte Pflasterfläche vollständig abdeckt. Wenn die Abdeckanordnung mehrteilig ausgeführt ist, kann bei der Applikation zunächst ein erster Abdeckstreifen von der Fläche gelöst werden, der so freigelegte Haftflächenbereich an der Haut fixiert und anschließend der oder die verbleibenden Abdeckstreifen von der Fläche gelöste und die Fixierung vollendet werden.

Nachstehend wird die Erfindung unter Bezugnahme auf die Zeichnung, auf die hinsichtlich aller erfindungswesentlichen und in der Beschreibung nicht weiter herausgestellten Einzelheiten ausdrücklich verwiesen wird, erläutert. In der Zeichnung zeigt:
- Fig. 1: ein Kanülenfixierpflaster gemäß einer ersten Ausführungsform der Erfindung und
- Fig. 2: ein Kanülenfixierpflaster gemäß einer zweiten Ausführungsform der Erfindung.

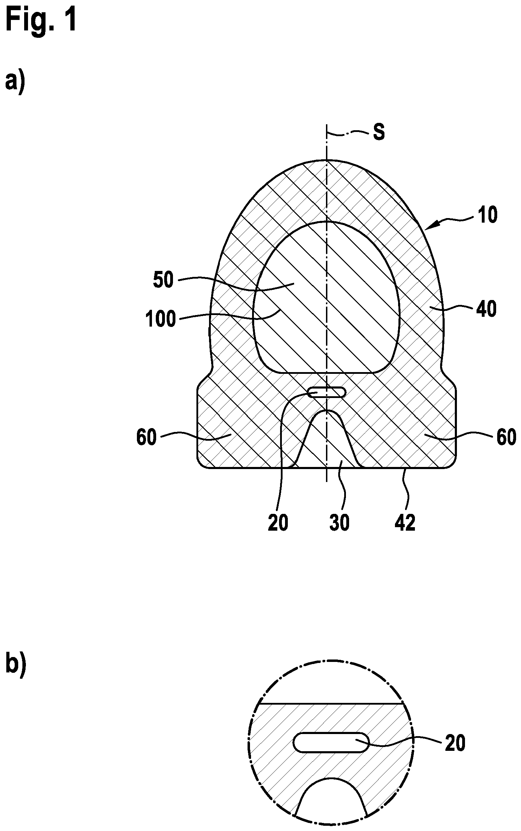

In Fig. 1a) ist eine Draufsicht auf das erfindungsgemäße Kanülenfixierpflaster in Richtung auf dessen Barrierelage dargestellt. Fig. 1b) zeigt eine Detaildarstellung des Kanülenfixierpflasters gemäß 1a). Das in Fig. 1 dargestellte Kanülenfixierpflaster umfasst eine insgesamt mit 10 bezeichnete und in der Zeichnung schraffiert dargestellte Trägeranordnung sowie eine als transparente Polyurethanfolie ausgeführte Barrierelage 100, welche die Trägeranordnung 10 vollständig überdeckt.

Die Trägeranordnung 10 weist einen eine von der Barrierelage 100 überdeckte Beobachtungsöffnung 50 umlaufenden Rahmen 40 auf. Mit Abstand von der Beobachtungsöffnung 50 ist zwischen der Beobachtungsöffnung 50 und einem Rand der Trägeranordnung 10 ein Aufnahmeschlitz 20 vorgesehen, der die Trägeranordnung durchsetzt und von der Barrierelage 100 überdeckt wird. Auf der der Beobachtungsöffnung 50 abgewandten Seite des Aufnahmeschlitzes 20 ist eine sich ausgehend von einem Rand 22 der Trägeranordnung 10 in Richtung auf den Aufnahmeschlitz 20 erstreckende Einbuchtung vorgesehen, welche sich ausgehend von dem Rand 42 der Trägeranordnung 10 in Richtung auf den Aufnahmeschlitz 20 verjüngt.

Zwischen dem dem Aufnahmeschlitz 20 zugewandten Ende der Einbuchtung 30 und dem Aufnahmeschlitz ist ein Materialstreifen der Trägeranordnung 10 mit einer Breite von etwa 3 mm freigelassen. Ebenso ist zwischen dem Aufnahmeschlitz 20 und der Beobachtungsöffnung 50 ein Abschnitt der Trägeranordnung 10 bzw. des Rahmens 40 mit einer Breite von etwa 3 mm vorgesehen. Die Gesamtanordnung des Kanülenfixierpflasters ist etwa spiegelsymmetrisch bezüglich einer senkrecht zur Längsrichtung des Aufnahmeschlitzes 20 verlaufenden Symmetrieebene S ausgeführt. Dabei ist die Breite der Trägeranordnung 10 im Bereich der Beobachtungsöffnung 50 in einer senkrecht zur Symmetrieebene S verlaufenden Ebene geringer als die Breite der Trägeranordnung im Bereich des Aufnahmeschlitzes 20 bzw. der Einbuchtung 30. Dadurch werden im Bereich des Aufnahmeschlitzes 20 bzw. der Einbuchtung 30 Fixierflügel 60 gebildet, welche das Fixieren des Fixierpflasters begünstigen.

Auf der der Barrierelage 100 abgewandten Begrenzungsfläche ist die Trägeranordnung 10 mit einem Haftmittel ausgestattet, welches vor der Applikation von einer mehrteiligen Abdeckanordnung aus Silikonpapier abgedeckt wird. Die Barrierelage 100 ist zumindest im Bereich der Beobachtungsöffnung 50 und des Aufnahmeschlitzes 20 auf ihrer der Trägeranordnung 10 zugewandten Begrenzungsfläche ebenfalls mit einem Haftmittel ausgestattet, welche das Fixieren des Kanülenfixierpflasters insgesamt begünstigt. Die Barrierelage 100 ist im Übrigen haftend mit der Trägeranordnung 10 verbunden.

Bei der in der Zeichnung dargestellten Ausführungsform der Erfindung beträgt die Länge des Aufnahmeschlitzes insgesamt 8 mm, die Breite des Schlitzes in einer senkrecht zur Längsrichtung verlaufenden Richtung beträgt 2 mm. Die Breite des Fixierpflasters im Bereich der Beobachtungsöffnung beträgt maximal 50 mm, während sie im Bereich der Einbuchtung 20 insgesamt 55 mm beträgt. Das dem Aufnahmeschlitz 20 zugewandte Ende der Einbuchtung 30 ist abgerundet ausgeführt, um so einem Einreißen des Fixierpflasters im Bereich des Scheitels der Einbuchtung 30 entgegenzuwirken.

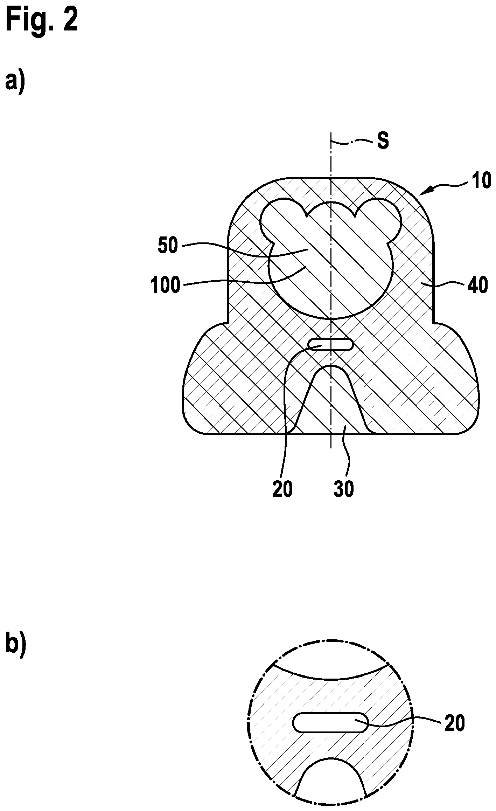

Die in Fig. 2 dargestellte Ausführungsform der Erfindung unterscheidet sich im Wesentlichen von der in Fig. 1 erläuterten Ausführungsform dadurch, dass die Beobachtungsöffnung 50 einem Tierkopf nachgebildet ist und die Abmessungen des Kanülenfixierpflasters insgesamt geringer sind. Allerdings sind die Abmessungen des Aufnahmeschlitzes 20 ebenso wie die Abmessungen der Einbuchtung 30 im Vergleich zu der in Fig. 1 dargestellten Ausführungsform der Erfindung im Wesentlichen unverändert. Das in Fig. 2 dargestellte Kanülenfixierpflaster ist zur Behandlung von Kindern vorgesehen. Allerdings tragen die Abmessungen des Aufnahmeschlitzes 20 und der Einbuchtung 30 dem Umstand Rechnung, dass Kanülenschlauch und Anfasshilfe bei Kindern dieselben Abmessungen haben wie bei der Behandlung von Erwachsenen. Es wird auch darauf hingewiesen, dass sich die Fixierflügel in Richtung auf den parallel zu dem Aufnahmeschlitz 20 verlaufenden Rand des Fixierpflasters insgesamt verbreitern, um so ausreichend Haftfläche neben der Einbuchtung 30 mit im Vergleich zu für die Behandlung von Erwachsenen unveränderten Abmessungen bereitzustellen.

Die Erfindung ist nicht auf die anhand der Zeichnung erläuterten Ausführungsbeispiele beschränkt. Vielmehr ist auch an den Einsatz von Kanülenfixierpflastern ohne Beobachtungsöffnung und/oder mit geänderten Abmessungen gedacht. Anstelle der sich verjüngenden Einbuchtung kann auch eine rechteckförmige Einbuchtung vorgesehen sein. Die Breite des Kanülenfixierpflasters im Bereich der Einbuchtung kann vergrößert werden, um so eine zuverlässigere Fixierung zu erreichen.

Die Beobachtungsfenster können grundsätzlich in beliebiger Form ausgeführt sein. Es ist allerdings darauf zu achten, dass die Breite des Rahmenabschnitts zwischen Beobachtungsfenster und Aufnahmeschlitz bzw. zwischen Aufnahmeschlitz und Einbuchtung ausreichend groß ist, um Zugkräfte aufnehmen zu können und ein unbeabsichtigtes Einreißen zu vermeiden.

Die Trägeranordnung ist bei den anhand der Zeichnung erläuterten Ausführungsbeispielen aus einer mit einem Haftmittel ausgestatteten Vliesschicht ausgeführt, sie kann auch als Folienschicht ausgeführt sein. Eine Vliesschicht hat allerdings den Vorteil, dass sie aus der Kanüleneinstichstelle austretendes Exsudat aufzunehmen vermag.

## Patentansprüche

1. Pflaster zum Fixieren von Kanülen mit einer eine Haftfläche aufweisenden Trägeranordnung (10) für eine auf der der Haftfläche abgewandten Seite der Trägeranordnung angeordnete Barrierelage (100), wobei die Trägeranordnung mittels der Haftfläche an der die Kanüleneinstichstelle umgebenden Haut fixierbar ist und die Barrierelage (100) die Migration von Bakterien hemmt und die Kanüleneinstichstelle überdeckt, wobei die Trägeranordnung (10) eine von der Barrierelage (100) überdeckte und zum Aufnehmen einer stegförmigen Anfasshilfe der Kanüle ausgelegte, die Trägeranordnung durchsetzende Aufnahme aufweist, **dadurch gekennzeichnet, dass** die Aufnahme (20) als geradlinig verlaufender Aufnahmeschlitz (20) in der als Vliesschicht ausgeführten Trägeranordnung (10) ausgeführt ist, wobei die Breite des Aufnahmeschlitzes in einer senkrecht zu dessen Längsrichtung verlaufenden Richtung weniger als 35 % der Schlitzlänge beträgt die Trägeranordnung (10) eine sich ausgehend von einem parallel zur Längsrichtung des Aufnahmeschlitzes (20) verlaufenden Rand (42) erstreckende Einbuchtung (30) aufweist, die sich ausgehend von einer die Länge des Aufnahmeschlitzes (20) übersteigenden Breite in Richtung auf den Aufnahmeschlitz verjüngt und an ihrem dem Aufnahmeschlitz zugewandten Ende eine geringere Breite aufweist als der Aufnahmeschlitz mit einer von der Trägeranordnung (10) nach Art eines Rahmens umlaufenen und durch einen Rahmenabschnitt der Trägeranordnung (10) von dem Aufnahmeschlitz (20) getrennten, auf der der Haftfläche abgewandten Seite der Trägeranordnung (10) von der Barrierelage (100) überdeckten Beobachtungsöffnung (50).

2. Pflaster nach Anspruch 1, **dadurch gekennzeichnet, dass** die Länge des Aufnahmeschlitzes weniger als 50 % der Breite der Trägeranordnung in Längsrichtung des Schlitzes und/oder mehr als 7 % der Breite der Trägeranordnung im Bereich des Schlitzes beträgt.

3. Pflaster nach einem der vorhergehenden Ansprüche, **dadurch gekennzeichnet, dass** die Breite des Schlitzes in einer senkrecht zu dessen Längsrichtung verlaufenden Richtung mehr als 10 % der Schlitzlänge beträgt.

4. Pflaster nach einem der vorhergehenden Ansprüche, **dadurch gekennzeichnet, dass** die Beobachtungsöffnung (50) in einer senkrecht zum Aufnahmeschlitz (20) verlaufenden Richtung von dem Aufnahmeschlitz (20) beabstandet ist, wobei der Aufnahmeschlitz (20) zwischen der Beobachtungsöffnung (50) und der Einbuchtung (30) angeordnet ist.

5. Pflaster nach einem der vorhergehenden Ansprüche, **dadurch gekennzeichnet, dass** die Breite der Trägeranordnung (10) im Bereich der Beobachtungsöffnung (50) geringer ist als die Breite der Trägeranordnung (10) im Bereich der Einbuchtung (30) und/oder der Aufnahme (20).

6. Pflaster nach einem der vorhergehenden Ansprüche, **dadurch gekennzeichnet, dass** die Trägeranordnung (10) insgesamt etwa spiegelsymmetrisch bezüglich einer etwa senkrecht zum Aufnahmeschlitz (20) verlaufenden Symmetrieebene (S) ausgeführt ist.

7. Pflaster nach einem der vorhergehenden Ansprüche, **dadurch gekennzeichnet, dass** die Barrierelage (100) eine transparente Polyurethanfolie aufweist.

8. Pflaster nach einem der vorhergehenden Ansprüche, **dadurch gekennzeichnet, dass** die Barrierelage (100) zumindest im Bereich der Aufnahme (20) und/oder der Beobachtungsöffnung (50) mit einem das Fixieren des Pflasters an der Haut und/oder der Anfasshilfe fördernden Haftmittel ausgestattet ist.

9. Pflaster nach einem der vorhergehenden Ansprüche, **gekennzeichnet durch** eine lösbar an der Haftfläche angeordnete mehrteilige Abdeckanordnung.

## Claims

1. A plaster for fixing cannulas comprising a support structure (10), having an adhesive surface, for a barrier layer (100) arranged on the side of the support structure (10) facing away from the adhesive surface, wherein the support structure (10) is fixable to the skin surrounding the cannula puncture site using the adhesive surface and the barrier layer (100) impedes the migration of bacteria and covers the cannula puncture site, wherein the support structure (10) has a cavity covered by the barrier layer (100) and configured for holding in place a ridge-shaped gripping aid on the cannula, which cavity penetrates the support structure, **characterised in that** the cavity (20) is configured as a cavity slot (20), running in a straight line, in the support structure (10) configured as a non-woven fabric layer, wherein the width of the cavity slot is less than 35% of the length of the slot in a direction perpendicular to its longitudinal direction; **in that** the support structure (10) has a notch (30) extending from an edge (42) running parallel to the longitudinal direction of the cavity slot (20), said notch tapering from a width exceeding the length of the cavity slot (20) as it proceeds towards the cavity slot and having at its end proximate to the cavity slot a smaller width than the cavity slot; and **in that** the support structure has an inspection opening (50), surrounded by the support structure (10) in the manner of a frame and separated from the cavity (20) by a frame section of the support structure (10), which inspection opening is covered by the barrier layer (100) on the side of the support structure (10) facing away from the adhesive surface.

2. The plaster according to claim 1, **characterised in that** the length of the cavity slot is less than 50% of the width of the support structure in the longitudinal direction of the slot and/or more than 7% of the width of the support structure proximate to the slot.

3. The plaster according to one of the preceding claims, **characterised in that** the width of the slot in a direction perpendicular to its longitudinal direction is more than 10% of the length of the slot.

4. The plaster according to one of the preceding claims, **characterised in that** the inspection opening (50) is located at a distance from the cavity slot (20) in a direction perpendicular to the cavity slot (20), wherein the cavity slot (20) is located between the inspection opening (50) and the notch (30).

5. The plaster according to one of the preceding claims, **characterised in that** the width of the support structure (10) is smaller proximate to the inspection opening (50) than the width of the support structure (10) proximate to the notch (30) and/or the cavity (20).

6. The plaster according to one of the preceding claims, **characterised in that** the support structure (10) is overall configured approximately in mirror symmetry in relation to a symmetry plane (S) running approximately perpendicular to the cavity slot (20).

7. The plaster according to one of the preceding claims, **characterised in that** the barrier layer (100) has a transparent polyurethane film.

8. The plaster according to one of the preceding claims, **characterised in that** the barrier layer (100) is provided, at least proximate to the cavity (20) and/or the inspection opening (50), with an adhesive promoting the fixing of the plaster to the skin and/or gripping aid.

9. The plaster according to one of the preceding claims, **characterised by** a covering arrangement consisting of multiple pieces and arranged detachably on the adhesive surface.

## Revendications

1. Pansement de fixation de canules comportant une structure de support (10) présentant une surface adhésive et destinée à une couche barrière (100) disposée sur la face de la structure de support (10) qui est détournée de la surface adhésive, ladite structure de support (10) pouvant être fixée sur la peau entourant le point de piqûre de canule au moyen de ladite surface adhésive et ladite couche barrière (100) empêchant la migration de bactéries et recouvrant ledit point de piqûre de canule, ladite structure de support (10) présentant un logement traversant ladite structure de support et recouvert par ladite couche barrière (100), conçu pour loger un auxiliaire de préhension en forme de nervure de la canule, **caractérisé en ce que** ledit logement (20) est conçu sous la forme d'une fente de logement (20) rectiligne dans la structure de support (10) conçu sous la forme d'un non-tissé ; la largeur de ladite fente de logement, dans un sens perpendiculaire à son sens longitudinal, faisant moins de 35 % de la longueur de ladite fente ; et **en ce que** la structure de support (10) présente une échancrure (30) qui part d'un bord (42) parallèle au sens longitudinal de la fente de logement (20) et qui va en s'amenuisant en direction de la fente de logement en présentant une largeur de départ qui dépasse la longueur de la fente de logement (20) et en se terminant à proximité de la fente de logement par une largeur plus petite que la fente de logement, et **en ce que** le pansement présente une ouverture d'observation (50) entourée par ladite structure de support (10) à la manière d'un cadre et séparée dudit logement (20) par une section d'encadrement de la structure de support (10), laquelle ouverture d'observation est recouverte par la couche barrière (100) sur la face de la structure de support (10) qui est détournée de la surface adhésive.

2. Pansement selon la revendication 1, **caractérisé en ce que** la longueur de la fente de logement fait moins de 50 % de la largeur de la structure de support, dans le sens longitudinal de la fente, et/ou plus de 7 % de la largeur de la structure de support située à proximité de ladite fente.

3. Pansement selon l'une des revendications précédentes, **caractérisé en ce que** la largeur de la fente, dans un sens perpendiculaire à son sens longitudinal, fait plus de 10 % de la longueur de la fente.

4. Pansement selon l'une des revendications précédentes, **caractérisé en ce que** l'ouverture d'observation (50) est espacée de la fente de logement (20) dans un sens perpendiculaire à ladite fente de logement (20), ladite fente de logement (20) étant située entre ladite ouverture d'observation (50) et ladite échancrure (30).

5. Pansement selon l'une des revendications précédentes, **caractérisé en ce que** la largeur de la structure de support (10), à proximité de l'ouverture d'observation (50), est inférieure à la largeur de la structure de support (10) à proximité de l'échancrure (30) et/ou du logement (20).

6. Pansement selon l'une des revendications précédentes, **caractérisé en ce que** la structure de support (10) est conçue pour être globalement sensiblement symétrique par rapport à un plan de symétrie (S) qui est sensiblement perpendiculaire à la fente de logement (20).

7. Pansement selon l'une des revendications précédentes, **caractérisé en ce que** la couche barrière (100) présente une feuille de polyuréthane transparente.

8. Pansement selon l'une des revendications précédentes, **caractérisé en ce que** la couche barrière (100) est dotée, au moins à proximité du logement (20) et/ou de l'ouverture d'observation (50), d'un adhésif facilitant la fixation du pansement à la peau et/ou à l'auxiliaire de préhension.

9. Pansement selon l'une des revendications précédentes, **caractérisé par** une structure protectrice en plusieurs pièces disposée détachable sur la surface adhésive.
